# EUROPEAN PATENT SPECIFICATION

(11) **EP 2 793 783 B1**
(45) Date of publication and mention of the grant of the patent: **22.01.2020**
(21) Application number: 11878017.0
(22) Date of filing: 21.12.2011
(51) Int. Cl.: G08B 21/18, H04M 1/725, A61F 13/84, A61F 13/42, H04M 1/21

(54) **METHOD, MONITORING SYSTEM AND COMPUTER PROGRAM FOR MONITORING USE OF AN ABSORBENT PRODUCT**
VERFAHREN, ÜBERWACHUNGSSYSTEM UND COMPUTERPROGRAMM ZUR ÜBERWACHUNG DER VERWENDUNG EINES SAUGFÄHIGEN PRODUKTS
PROCÉDÉ, SYSTÈME DE SURVEILLANCE ET PROGRAMME INFORMATIQUE POUR SURVEILLER L'UTILISATION D'UN PRODUIT ABSORBANT

(43) Date of publication of application: 29.10.2014
(73) Proprietor: Essity Hygiene and Health Aktiebolag, 405 03 Göteborg (SE)
(72) Inventor: CARNEY, Joshua, 405 03 Göteborg (SE)
(74) Representative: Zacco Sweden AB
(86) International application number: PCT/SE2011/051558
(87) International publication number: WO 2013/095226

(56) References cited:
- WO-A1-2011/054045
- WO-A1-2011/126497
- JP-A- 2002 107 361
- JP-A- 2002 113 008
- JP-A- 2003 111 797
- JP-A- 2007 167 264
- US-A1- 2004 100 376
- US-A1- 2004 220 538
- US-A1- 2007 142 799
- US-A1- 2007 270 774
- US-A1- 2007 270 774
- US-A1- 2008 058 740
- US-A1- 2011 015 496
- US-A1- 2011 046 571

## Description

### TECHNICAL FIELD

The present invention relates to a method and mobile monitoring system for monitoring use of an absorbent article. It also relates to a mobile device of such a monitoring system and a computer program for causing the mobile device and the monitoring system to carry out the method.

### BACKGROUND

Urinary and/or faecal incontinence causes many people to use various types of absorbent products, such as incontinence pads, diapers etc.

In their everyday life, those affected by incontinence are often anxious that people in their immediate surroundings can scent the odour of urine or faeces escaping from the absorbent products.

Absorbent products are often capable of absorbing and retaining a certain amount of urine and/or faeces but too late change of the absorbent product may cause the odours to escape and be perceived by people in the surroundings. To many people suffering from incontinence, this is a huge problem often causing feelings of shame and humiliation and sometimes even social withdrawal and isolation.

There are several known arrangements for monitoring urinary or faecal voiding in order to allow for timely change of absorbent products.

For example, there are diapers including wetness sensors capable of triggering an alarm if the number of discrete insults has reached a certain insult limit. Such a diaper is disclosed in US 2009/0062758 A1. Another incontinence management system for monitoring wetness in absorbent articles is disclosed in US 2011/0263952.Still further examples of incontinence management system having an absorbent article with integrated wetness sensor are known from US 2007/0270774 A1 and WO 2011/054045 A1.

A monitoring system that uses smell sensors to detect urinary or faecal voiding by dementia patients is disclosed in JP2007167264 A.

However, known solutions for monitoring use of absorbent articles often involve complex and expensive products and/or monitoring systems that are not readily available to the public. Furthermore, known monitoring systems are not discrete enough to be used by active and social people in their everyday life.

### SUMMARY OF THE INVENTION

It is an object of the present invention to solve or at least mitigate one or more of the above mentioned problems.

In particular, it is an object of the present invention to provide cost efficient and readily available means for monitoring use of absorbent products.

Another object of the invention is to provide means for preventing too late change of absorbent products.

Yet another object of the invention is to provide means for monitoring use of absorbent products that are discrete and can be used by people suffering from incontinence in any social situation.

These and other objects are achieved by a method for monitoring use of an absorbent product as defined in claim 1.

The objects are also achieved by a mobile monitoring system for monitoring use of an absorbent product worn by a user as defined in claim 8.

By carrying the odour sensor device, e.g. in a pocket of clothing, the user can be notified via a mobile device, such as a conventional mobile phone, that urinary and/or faecal voiding has taken place and that it may be desirable to change the absorbent product. The use of a small-sized odour sensor device and a conventional mobile device, such as a mobile phone, makes the monitoring system according to the invention mobile, discrete and easy to use in everyday life situations.

Product-related information herein means information related to the use of the absorbent product. The product-related information may comprise any of or any combination of:
- an indication that no change of product is necessary,
- an indication that urinary and/or faecal voiding has taken place and that it may be advisable to change the product,
- a recommendation to change the product,
- a recommendation to change the product before a certain time,
- a recommendation to exchange the product for another absorbent product having higher or lower capacity than the currently worn product.

The content of the product-related information and additionally also the way the product-related information is provided to the user is determined by the mobile device based on the signal received from the odour sensor device.

The odour sensor device comprises an odour sensor, such as a nanosensor or an electrochemical gas sensor. The odour sensor is configured to detect concentrations of the at least one chemical indicative of presence of urine and/or faeces in the absorbent product that are well below concentrations perceivable by humans. In this way, the user can be provided with the product-related information well before the odour of urine or faeces can be scented by persons in the immediate surroundings of the user.

The odour sensor may be configured to detect any type of airborne molecules indicative of the presence of urine and/or faeces. Examples of chemicals that are indicative of the presence of urine and detectable by suitably sized odour sensors of today are ammonia, amines, sulphides and ketones. Corresponding examples of chemicals indicative of the presence of faeces are hydro dioxide, indole, skatole, thiols (sulfer) and hydrogen sulphide.

Furthermore, the odour sensor device comprises a communication unit providing for communication with the mobile device. Preferably, the communication unit of the odour sensor device and the mobile device are configured for wireless communication with each other. To this end, the communication unit of the odour sensor device may be configured for bluetooth-based communication with the mobile device.

In other embodiments, the odour sensor device is designed as a mobile device adapter capable of being detachably connected to the mobile device. To this end, the odour sensor device may comprise a connector for detachable connection to a standard connector of a mobile phone, and the communication unit of the odour sensor device may be configured to communicate with the mobile phone through said connector. For example, the odour sensor device may be realised as a mobile device adapter adapted for detachable connection to an iPhone or an iPad. The odour sensor device connector may in this case be a 30-pin male connector.

When adapted for wireless communication with the mobile device, the odour sensor device is preferably designed to be carried in a pocket of clothing of the user, e.g. a trouser pocket. It may also comprise attachment means, such as a clip, allowing it to be attached to the clothing, e.g. a belt, of the user. When realised in form of an adapter that is detachably connectable to a mobile phone, the odour sensor device is preferably sized such that the mobile phone and the odour sensor device can be carried in a pocket of clothing of the user when connected to each other, or in a case for mobile phones, attachable to a belt of the user.

Preferably, the odour sensor device is configured to generate the signal to the mobile device in dependence of the concentration of the at least one detected chemical, meaning that the signal is indicative of the concentration of the at least one detected chemical. The mobile device may in turn be configured to estimate an amount of urine and/or faeces present in the absorbent product based on the signal, and to provide the product-related information based on said estimation.

The method further comprises the steps of obtaining capacity information related to the capacity of the absorbent product, and providing the product-related information based on both the signal received from the odour sensor device and this capacity information. This allows the product-related information to be based on a comparison between an estimated amount of urine and/or faeces present in the absorbent product and the capacity of the product, which in turn allows the product-related information to comprise well-founded recommendations as to the need of changing the product. Preferably, the product-related information comprises a recommendation to change the absorbent product before the estimated amount of urine and/or faeces in the product exceeds the amount of urine and/or faeces that can be absorbed and/or retained by the product.

To this end, the method may comprise the steps of determining a capacity parameter indicative of the amount of urine and/or faeces that can be absorbed by the product based on said capacity information, and a voiding parameter indicative of the amount of urine and/or faeces present in the absorbent product based on the signal received from the odour sensor device. The product-related information is then provided based on a comparison between the capacity parameter and the voiding parameter. If the comparison shows that the amount present in product is close to the capacity of the product, the product-related information can include a recommendation to change the product as soon as possible. If, however, the amount present in the product is well below the capacity of the product, the product-related information may comprise an indication that no change of product is required at the moment.

According to one embodiment of the invention, the mobile device is configured to determine a maximum threshold value for the concentration of the at least one chemical based on the capacity information, and to compare the detected chemical concentration as indicated by the signal received from the odour sensor device with the maximum threshold value. If the maximum threshold value is exceeded, the mobile device notifies the user that the absorbent product should be changed as soon as possible, and if the detected chemical concentration is well below said maximum threshold value, the mobile device may notify the user that no change is required at the moment.

According to another embodiment of the invention, the mobile device is configured to determine a maximum number of insults of urine and/or faeces that can be absorbed and/or retained by the absorbent product, based on the capacity information. In this embodiment, the mobile device is further configured to estimate the number of insults of urine and/or faeces voided by the user, based on the signal received from the odour sensor device. The product-related information to be provided to the user may then be selected by the mobile device based on a comparison between said estimated number of insults and said maximum number of insults.

The number of insults voided by the user may be estimated based on the concentration of the at least one detected chemical. To this end, the mobile device may be configured to determine a plurality of ranges of concentration for the at least one chemical, each range corresponding to a certain number of insults, and to determine how many insults the detected concentration corresponds to by determining within which range the detected concentration falls.

The mobile device may also be configured to use the rate of change of the concentration of the at least one detected chemical to determine the number of insults voided by the user. For example, it may be configured to compare the rate of change of the detected concentration as indicated by signals received from the odour sensor device with an insult threshold value, and to assume that an insult has occurred if the insult threshold value is exceeded. By keeping track of the number of times the insult threshold value is exceeded, the mobile device can count the number of insults of urine and/or faeces voided by the user and compare them with the above mentioned maximum number of insults.

Preferably, the mobile device is further configured to predict a future need of changing the absorbent product based on the concentration of the at least one detected chemical, and to display product-related information on the mobile device indicative of this future need. For example, the mobile device may be configured to display a future point in time before which the product should be changed. In order to predict the future need of changing the product, the mobile device may be configured to determine a trend for the detected concentration of the at least one chemical based on the signals received from the odour sensor device, and to predict a future point in time at which the concentration exceeds a maximum threshold value. The maximum threshold value is preferably determined by the mobile device based on the capacity of the product as discussed above.

The mobile device may also be configured to determine a trend for the number of discrete insults voided by the user based on the timing of detected insults, and to predict future insults based on this trend. The future need of changing the product can then be predicted by the mobile device by estimating when the number of insults voided by the user will exceed a maximum number of insults that can be absorbed and/or retained by the product, which maximum number of insults may be determined from the capacity information.

The capacity information related to the capacity of the absorbent product is typically used by the mobile device to determine a capacity parameter that can be compared with a voiding parameter indicative of the presence of urine and/or faeces in the absorbent product. As understood from the above discussion, the capacity parameter may be a maximum threshold value for the concentration of the at least one chemical detected by the odour sensor device, or a maximum number of discrete insults that can be absorbed and/or retained by the product. The voiding parameter may be the concentration of the at least one detected chemical or an estimated number of urinary and/or faecal insults by the user, estimated based on the magnitude or rate of change of said concentration.

The capacity information obtained by the mobile device and used to determine the capacity parameter may comprise information related to the type of the absorbent product, the absorbency level of the absorbent product, and/or the size of the absorbent product. The capacity information may be obtained by the mobile device from user input or from a product database stored on the mobile device or an application server to which the mobile device is connectable.

The mobile device may be any mobile device, i.e. portable device, capable of communicating with the odour sensor device and capable of providing the product-related information to the user in form of visual, audible and/or vibratory signals, such as a mobile phone, a personal digital assistant (PDA), a tablet computer or any other hand-held computing device. Preferably, the mobile device should have a size allowing it to be carried in a pocket of clothing of the user, or in a case that can be attached to the belt of the user.

The method according to the invention is preferably computer-implemented and performed by the mobile monitoring system through execution of a computer program stored on the mobile device. Preferably, this computer program is realised in form of an App that is downloadable to a storage medium of the mobile device. By allowing the method to be performed through execution of an App that may be downloaded into existing mobile devices, the method becomes readily available to anyone.

When executed by the mobile device, the computer program hence causes the mobile device to carry out a method comprising the steps of:- obtaining capacity information related to the capacity of the absorbent product, wherein the absorbent product is a male or female incontinence protector, a sanitary pad, a diaper with tape fastener, a pant diaper or a belted diaper,
- receiving, from an odour sensor device capable of being carried by the user, a signal indicating that the odour sensor device has detected at least one chemical indicative of a presence of urine and/or faeces in the product, wherein said signal is indicative of the concentration of the at least one detected chemical, and wherein the odour sensor device is configured to be carried in a pocket of clothing of the user, or to be attached to the clothing of the user, or wherein the odour sensor device is realised in form of an adapter that is detachably connectable to the mobile device,
- providing product-related information to the user based on both the signal received from the odour sensor device and said capacity information, and adjusting the content of the product-related information based on said concentration.

In addition, the computer program may be configured to cause the mobile device or the odour sensor device to carry out any additional method steps described above.

The invention also provides a computer program product comprising a non-volatile memory for storing computer-readable instructions, wherein the above mentioned computer program is encoded on said non-volatile memory.

Furthermore, the invention provides a mobile device for monitoring use of an absorbent product worn by a user. The mobile device comprises a processor, a communication unit for communicating with the odour sensor device, and a storage medium for storing computer programs executable by said processor, wherein the mobile device comprises means for performing the method when executing the computer program defined above.

### BRIEF DESCRIPTION OF THE DRAWINGS

Fig. 1 illustrates a mobile monitoring system for monitoring use of an absorbent product according to an embodiment of the invention.
Fig. 2 illustrates a mobile monitoring system for monitoring use of an absorbent product according to another embodiment of the invention.
Figs. 3A and 3B illustrate the mobile monitoring systems of Figs. 1 and 2 when carried by a user of an absorbent product.
Fig. 4 is a flow chart illustrating the basic principles of a method for monitoring use of an absorbent article.
Fig. 5 is a flow chart illustrating a refined embodiment of the method according to the invention.

### DETAILED DESCRIPTION

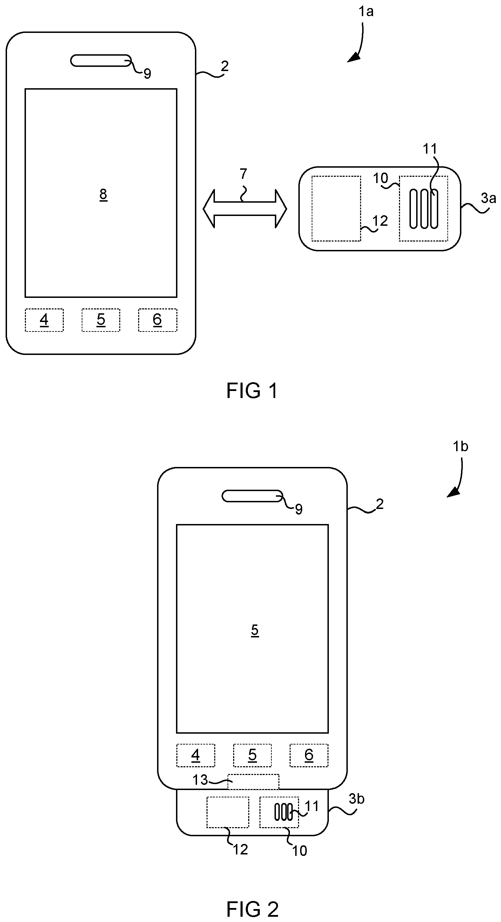

Fig. 1 illustrates a mobile, i.e. a portable, monitoring system 1a for monitoring use of an absorbent product. The monitoring system 1a comprises a mobile device 2 and an odour sensor device 3a which cooperate to provide a user of the absorbent product with information related to the use of the product. The absorbent product referred to herein is an absorbent personal hygiene article, including but not limited to male or female incontinence protectors, sanitary pads, diapers with tape fastener, pant diapers and belted diapers.

The mobile device 2 is a mobile phone in form of what is often referred to as a smartphone but it should be appreciated that the mobile device according to the invention may be any type of hand-held computing device, such as a personal digital assistant (PDA) or a tablet computer, devised and configured as set forth below.

The mobile device 2 comprises a processor 4 for processing data. The data to be processed may be received from the odour sensor device 3a, network nodes to which the mobile device 1 is communicatively connectable, or it may be stored on a digital storage medium 5 of the mobile device, which storage medium is accessible by the processor 4.

The mobile device 2 further comprises a communication unit 6 for communicating with the odour sensor device 3a over a wireless communication interface 7, such as a bluetooth interface. The communication unit 6 is preferably also configured to allow the mobile device 2 to communicate with application servers on one or more networks, such as the Internet.

The mobile device 2 is further seen to comprise a display 8 for displaying information to a user, and, if realised in form of a touch-display, also for receiving information from the user in form of user input. The mobile device 2 may also comprise other means for receiving user input, such as buttons, microphones, etc. Furthermore, the mobile device 2 comprises a loudspeaker 9 for outputting sound signals to the user.

The odour sensor device 3a is sized to be carried in a pocket of clothing of the user, and preferably to be carried in the pocket of a pair of trousers or a skirt. The odour sensor device 3a may further comprise attachment means (not shown), such as a clip, for attachment to the clothing of the user, e.g. to a belt.

The odour sensor device 3a comprises an odour sensor 10, indicated in dashed lines, comprising one or more gas inlets 11 through which the gas to be analysed can enter the odour sensor. The odour sensor 10 is configured to detect at least one airborne chemical indicative of a presence of urine and/or faeces in the absorbent product. Preferably the odour sensor 10 is configured to detect a plurality of airborne chemicals indicative of the presence of urine and/or faeces. To this end, the odour sensor 10 may comprise a plurality of sensor elements configured to detect different molecules. The odour sensor 10 may be configured to detect chemicals including, but not limited to, ammonia, amines, sulphides and ketones, all indicative of the presence of urine, and hydro dioxide, indole, skatole, thiols (sulfer) and hydrogen sulphide, all indicative of the presence of faeces.

The odour sensor 10 of the odour sensor device 3a may include a porous silicon wafer sensor, sometimes referred to as a nanosensor. For example, it may be a carbon nanotube based sensor operable to monitor electrical changes in the sensor material of a nanotube. For instance when a molecule of nitrogen dioxide (NO₂) is present, it will strip an electron from the nanotube, which in turn causes the nanotube to be less conductive. If ammonia (NO₃) is present, it reacts with water vapour and donates an electron to the carbon nanotube, making it more conductive. By treating the nanotube with various coating materials, it can be made sensitive to certain molecules and immune to others.

According to another embodiment, the odour sensor 10 is an electrochemical gas sensor. In this type of sensor, the gas to be analysed diffuses into the sensor through the back of a porous membrane to a working electrode where it is oxidized or reduced. This electrochemical reaction results in an electric current that passes through an external circuit of the odour sensor. In addition to measuring, amplifying and performing other signal processing functions, the external circuit maintains the voltage across the sensor between the working electrode and a counter electrode for a two electrode cell, or between the working and reference electrodes for a three electrode cell. At the counter electrode an equal and opposite reaction occurs, such that if the working electrode is an oxidation, then the counter electrode is a reduction.

Moreover, the odour sensor device 3a comprises a communication unit 12 for communicating with the mobile device 2. When the odour sensor 10 detects a chemical indicative of presence of urine and/or faeces, it generates a signal which is transmitted to the mobile device 2 by the communication unit 12. Preferably, the odour sensor 10 is configured to generate a signal in dependence of the concentration of the detected chemical, and hence send a signal that is indicative of the concentration of the detected chemical to the mobile device 2.

Fig. 2 illustrates a mobile monitoring system 1b according to another embodiment of the invention.

The monitoring system 1b differs from the monitoring system 1a in Fig. 1 mainly in that it comprises an odour sensor device 3b which is designed as a mobile device adapter configured for detachable connection to the mobile device 2. The detachable connection is achieved via a connector 13 of the odour sensor device 3b, which connector 13 is adapted to a connection interface of the mobile device 2. In this embodiment, the connector 13 and additionally also the communication unit 12 may have to be adapted to the connection interface of the particular mobile device for which the odour sensor device 3b is intended. The odour sensor device 3b and the mobile device 2 are preferably sized such that they both fit into a standard-sized trouser pocket. However, the monitoring system 1b may also be attached to the clothing of the user. For example, it may be carried in a case adapted for attachment to a belt of the user.

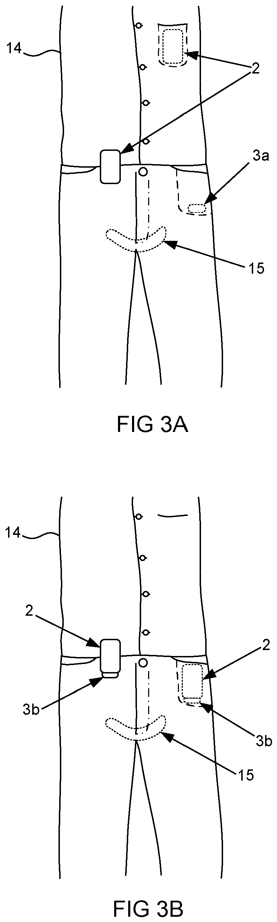

With simultaneous reference to Figs 1 and 2, Figs. 3A and 3B show examples of how the mobile monitoring systems 1a and 1b and their respective components can be carried by a user 14 in order to properly monitor the use of an absorbent product 15 in accordance with the inventive method described hereinafter.

It should be appreciated that some or all of the steps of this method is carried out by the mobile device 2 of the monitoring system 1a, 1b through execution of a computer program stored in the storage medium 5.

Preferably, this computer program is realised in form of a stand-alone application, meaning that no data has to be received from application servers residing on a network to which the mobile device 2 must be connected. However, the computer program may also be a client application of a distributed software solution further comprising a server-side application residing in an application server to which the mobile device is communicatively connectable. In this case, some of the method steps described below may be performed by the application server through execution of the server-side application.

In a preferred embodiment of the invention, the computer program stored on the mobile device 2 is realised in form of an App. An App, sometimes referred to as a mobile app or a mobile application, is a software application specifically designed to run on mobile devices such as smartphones and tablet computers. The App is downloadable into the storage medium 5 from a download server to which the mobile device 2 is connectable. The App may be adapted to a particular mobile operating system, such as Apple iOS, Google Android or Blackberry OS and distributed through known application distribution platforms.

It should thus be appreciated that "the App" hereinafter refers to the computer program stored on the storage medium 5 of the mobile device 2. The App can be executed by means of touching a particular icon displayed on the display 8 of the mobile device 2.

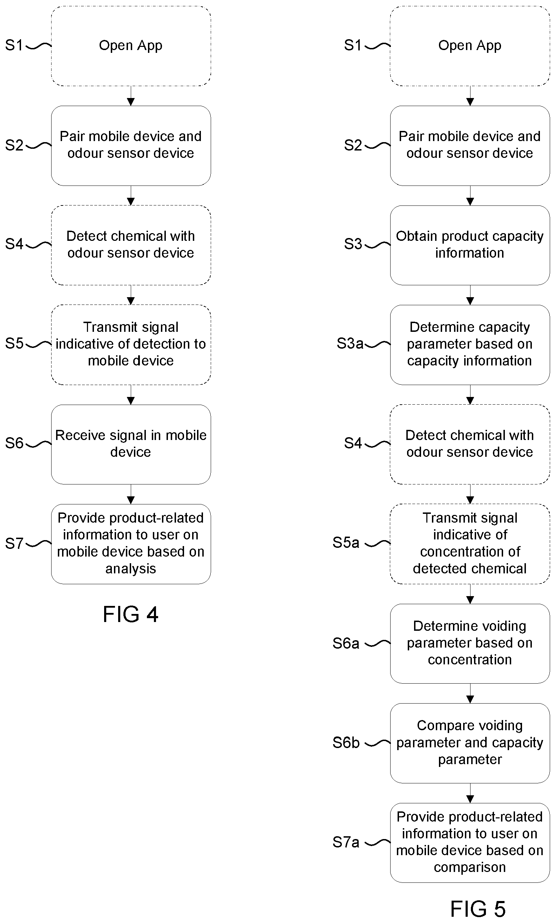

Figs. 4 to 5 are flowcharts illustrating different aspects of the method for monitoring use of an absorbent product. In the description of these flowcharts, simultaneous reference will be made to previously described drawings. In the flowcharts of figures 4 and 5, boxes drawn with dash-dotted lines indicate method steps performed by the user 14, boxes drawn with dashed lines indicate method steps performed by the odour sensor device 3a, 3b, and boxes drawn with continuous lines indicate method steps performed by the mobile device 2. The method steps performed by the mobile device 2 and the odour sensor device 3a, 3b may all be caused by the App of the mobile device 2.

Fig. 4 illustrates the basic principles of the method.

In a first step, S1, the App is opened by the user 14 of the absorbent product 15.

In a subsequent step S3, the mobile device 2 is paired with the odour sensor device 3a, 3b through a wireless or wired pairing procedure, depending on the communication interface 7 between the mobile device 2 and the odour sensor device 3a, 3b. The odour sensor device 3a, 3b is then placed in a position where the odour sensor device 3a, 3b is capable of detecting airborne chemicals emitted from the absorbent product 15.

In step S4, the odour sensor device 3a, 3b detects at least one chemical indicative of a presence of urine and/or faeces in the absorbent product 15.

In step S5, the odour sensor device 3a, 3b transmits a signal indicative of the detection in step S4 to the mobile device 2. The signal may be a single signal that is generated by the odour sensor device 3a, 3b when the concentration of the at least one chemical exceeds a predetermined threshold value. Preferably, however, the odour sensor device 3a, 3b is configured to repeatedly transmit signals indicative of the current concentration of the at least one detected chemical to the mobile device 2. To this end, the odour sensor device 3a, 3b may be configured to broadcast the concentration signals at given time intervals. In other variants of the method, the mobile device 2 may be configured to send a request for the current concentration of the at least one chemical to the odour sensor device 3a, 3b, whereby the odour sensor device 3a, 3b is configured to reply to the request by transmitting the signal to the mobile device 2.

In step S6, the signal is received by the mobile device 2.

In step S7, mobile device 2 provides product-related information related to the use of the absorbent product 15 to the user 14, based on the signal received from the odour sensor device 3a, 3b.

In variants where the signal transmitted from the odour sensor device 3a, 3b to the mobile device 2 indicates that the concentration of the detected chemical exceeds a predetermined threshold value, the mobile device 2 may be configured to provide the product-related information to the user 14 without further analysis of the received signal. For example, if the threshold value is set to a concentration close to the concentration at which the chemical can be scented by humans, the mobile device 2 may be configured to simply provide the product-related information in form of a visual, aural and/or vibratory alarm indicating to the user 14 that the absorbent product 15 should be changed as soon as possible.

In variants where the signal indicates the actual concentration of the at least one detected chemical, the mobile device 2 may be configured to analyse the received concentration signal and adjust the content of the product-related information, and additionally also the way the product-related information is provided to the user 14, based on the concentration of the chemical.

The product-related information is preferably provided to the user 14 through visual, audible and/or vibratory signalling on the mobile device 2. Instead, or in addition, it may be provided on a communication device, such as another mobile device or a computer, with which the mobile device can communicate, for example a communication device of a caregiver of the user. In the latter scenario, the product-related information may be provided to the communication device e.g. in form of a text message (sms) or an e-mail

Fig. 5 illustrates a more refined embodiment of the inventive method. Here the method is seen to comprise an additional step S3 of obtaining capacity information related to the product's capacity to absorb urine and/or faeces. This step should be performed prior to detection of the chemical in step S4.

The capacity information may comprise any of, or any combination of the type of the absorbent product, the absorbency level of the absorbent product, and the size of the absorbent product. The capacity information may be obtained from user input and/or reception of information from a server-side application residing on an application server with which the mobile device 2 can communicate.

In a preferred embodiment, step S3 of obtaining the capacity information comprises a first step of obtaining information about the type of the absorbent product, i.e. information telling the App whether the absorbent product is an incontinence pad, a diaper, etc. To this end, the App is preferably configured to display a list of several product types on the display of the mobile device 2, and to obtain the product type information by having the user 14 indicating the correct alternative in the list of product types. It may further comprise a second step in which the App obtains information about the absorbency level of the absorbent product 15. Many types of absorbent products, e.g. incontinence pads, are available within a wide range of absorbency levels, and the product's capability to absorb and retain liquid and/or faeces may vary substantially between different absorbency levels. Typically, the absorbency level is the level of the product's absorption capacity on a predefined scale, which level and scale are indicated on the package of the absorbent product. Preferably, the App is configured to display a scale of absorbency levels corresponding to a scale of absorbency levels presented on the package of the absorbency product, and to obtain the absorbency level by having the user indicating the correct absorbency level on the displayed scale.

The App may also be configured to obtain the capacity information automatically through automatic identification of the absorbent product. The App may be configured to identify the product automatically by means of a camera and suitable image recognition software, an RFID reader or a barcode scanner of the mobile device 2, and to obtain the capacity information automatically from a product database stored locally on the mobile device 2 or a product database stored on the above mentioned application server. To this end, the absorbent product may be provided with means for facilitating automatic identification thereof, such as a barcode (e.g. a QR code) or an RFID tag.

After having obtained the capacity information in step S3, the mobile device 2 determines a capacity parameter in a following step S3a. The capacity parameter is indicative of the amount of urine and/or faeces that can be absorbed by the product 14 and is determined by the mobile device 2 based on the capacity information obtained in step S3.

In one embodiment, the mobile device 2 is configured to determine a maximum threshold value for the concentration of the at least one chemical as said capacity parameter. This embodiment will hereinafter be referred to as the concentration-capacity embodiment. Adjusting the maximum threshold value based on the capacity information allows the mobile device 2 to set higher maximum threshold values for absorbent products having high capacities, and lower maximum threshold values for absorbent products having lower capacities. Thereby, the mobile device 2 can give the user 14 well-founded recommendations as to the need of changing the product. No matter the capacity of the product, the threshold value for the at least one chemical is preferably set by the mobile device 2 to a value that is well below the concentration perceivable by humans in the immediate surroundings of the user 14.

In another embodiment, the mobile device 2 may be configured to determine a maximum number of insults of urine and/or faeces that can be absorbed by the absorbent product as said capacity parameter. This embodiment will hereinafter be referred to as the insult-capacity embodiment.

In yet other embodiments, the mobile device 2 is configured to retrieve the capacity parameter from a database of the App itself or a database of a server-side application with which the App can communicate, using one or more parameters identifying the product type, the absorbency level of the product, and/or the size of the product as input parameters in the database request.

In step S4, the detection procedure starts and the odour sensor device 3a, 3b analyses the gas entering the odour sensor 10 to detect the one or more chemicals that are indicative of a presence of urine and/or faeces in the absorbent product 14.

When at least one such chemical is detected, the odour sensor device 3a, 3b, in step S5a, generates a signal that indicates the concentration of the detected chemical and transmits the signal to the mobile device 2.

In step S6a, the mobile device 3 receives the signal from the odour sensor device 3a, 3b and determines a voiding parameter indicative of the amount of urine and/or faeces that are present in the absorbent product 14, based on the concentration of the detected chemical, as indicated by the signal.

In the concentration-capacity embodiment described above, the mobile device 2 may use the concentration of the detected chemical as voiding parameter.

In the insult-capacity embodiment described above, the mobile device 2 may be configured to estimate the number of insults of urine and/or faeces by the user, based on the signal received from the odour sensor device, and to use said number as voiding parameter. The number of insults by the user may be estimated by the mobile device 2 by determining a plurality of ranges of concentration for the at least one chemical detected by the odour sensor device 3a, 3b, wherein each range of concentration corresponds to a certain number of insults. The number of insults by the user may then be estimated by the mobile device 2 by determining within which range the concentration indicated by the signal falls.

To estimate the number of insults or urine and/or faeces by the user, the mobile device may also be configured to use the rate of change of the concentration of the at least one detected chemical. For example, it may be configured to determine the rate of change of the concentration from a plurality of signals received from the odour sensor device at different points in time. The mobile device 2 may further be configured to compare the so determined rate of change with a threshold value (the "insult threshold value"), and to assume that an insult has occurred if the determined rate of change exceeds the insult threshold value. By repeatedly carrying out these calculations and counting the number of times the insult threshold value is exceeded, the mobile device can count the number of insults of urine and/or faeces by the user and use said number as voiding parameter.

In step S6b, the mobile device 2 compares the capacity parameter determined in step S3a with the voiding parameter determined in step S6a.

In the concentration-capacity embodiment, this means that the maximum threshold value for the concentration of the detected chemical is compared with the currently detected concentration of the chemical, as indicated by the signal from the odour sensor device 3a, 3b, and in the insult-capacity embodiment it means that the maximum number of insults that can be absorbed by the product 14 is compared with the estimated number of insults by the user.

In the last step S7a, the mobile device 2 provides the product-related information to the user based on the result of the comparison made in step S6b. At least the content of the product-related information but preferably also the way the product-related information is provided to the user is selected by the mobile device 2 based on the result of the comparison.

According to another aspect of the invention, the method may involve prediction of a future need of changing the absorbent product 15, and display of information related to this future need on the mobile device 2. To this end, the mobile device 2 may be configured to determine a trend for the detected concentration of the at least one chemical from a plurality of signals received from the odour sensor device 3a, 3b, and to predict a future point in time at which the concentration exceeds a maximum threshold value. The maximum threshold value is preferably set to correspond to a concentration of the chemical that is not perceivable by humans in the surroundings of the user 14. The maximum threshold value may further be determined by the mobile device 2 based on the capacity of the product as discussed above.

The mobile device 2 may also be configured to determine a trend for the number of discrete insults or urine and/or faeces by the user based on the timing of detected insults, and to predict future insults based on this trend. The future need of changing the product 14 can then be predicted by the mobile device 2 by estimating when the number of insults by the user 14 will exceed a maximum number of insults that can be absorbed and/or retained by the product 15, which maximum number of insults may be determined from the capacity information as also discussed above.

The mobile device 2 may use the prediction of the future need of changing the absorbent product 15 in order to display recommendations as to the use of the product 15 to the user 14. For example, the mobile device 2 can display a time before which the product 15 has to be changed to prevent that the odour of urine and/or faeces is perceived by the user 14 and people in the immediate surroundings of the user, and/or a time at which the absorbent product 15 will be saturated, meaning that it will be incapable of absorbing more insults.

For some absorbent products, it may be desirable to distinguish between urinary and faecal insults by the user 14. This is of particular interest if the absorbent product 15 is a product for absorption of both urine and faeces, e.g. a diaper. In this case, the mobile device 2 may be configured to obtain capacity information indicative of the product's capacity to absorb and/or retain urine and faeces, respectively. It may further be configured to estimate both the amount of urine and the amount of faeces present in the absorbent product 15 based on the signals received from the odour sensor device 3a, 3b. To this end, the odour sensor device 3a, 3b may be configured to make a distinction between chemicals indicative of urine and chemicals indicative of faeces.

It should thus be appreciated that the method steps described above, e.g. the steps of determining and comparing capacity and voiding parameters, may be performed for chemicals indicative of the presence of urine and faeces, individually.

## Claims

1. A method for monitoring use of an absorbent product (15) worn by a user (14), **characterised in that** the absorbent product (15) is a male or female incontinence protector, a sanitary pad, a diaper with tape fastener, a pant diaper or a belted diaper, and **in that** the method comprises the steps of:
- obtaining (S3), by means of a mobile device (2), capacity information related to the capacity of the absorbent product (15),
- detecting (S4), by means of an odour sensor device (3a;3b) capable of being carried by the user (14) and configured to communicate with said mobile device (2) of the user, at least one chemical indicative of a presence of urine and/or faeces in the product, wherein the odour sensor device (3a;3b) is carried in a pocket of clothing of the user (14), or attached to the clothing of the user (14), or wherein the odour sensor device (3a, 3b) is realised in form of an adapter that is detachably connectable to the mobile device (2),
- transmitting (S5), from said odour sensor device (3a;3b) to said mobile device (2), a signal indicative of said detection wherein said signal is indicative of the concentration of the at least one detected chemical,
- receiving (S6), in said mobile device, the signal indicative of the detection,
- providing (S7), by means of said mobile device, product-related information based on both said signal received from the odour sensor device and said capacity information.

2. The method according to claim 1, further comprising the step of adjusting the content of the product-related information based on the concentration of the at least one detected chemical.

3. The method according to claim 1, further comprising the steps of:
- determining (S3a), based on said capacity information, a capacity parameter indicative of the amount of urine and/or faeces that can be absorbed and/or retained by the absorbent product,
- determining (S6a), based on said signal, a voiding parameter indicative of the amount of urine and/or faeces present in the absorbent product,
- comparing (S6b) the capacity parameter and the voiding parameter, and
- providing (S7a) said product-related information based on said comparison.

4. The method according to claim 3, wherein the capacity parameter is determined based on any of, or any combination of:
- the type of the absorbent product (15),
- the absorbency level of the absorbent product (15), and
- the size of the absorbent product (15).

5. The method according to claim 3 or 4, wherein the voiding parameter is determined based on the concentration of the at least one detected chemical.

6. The method according to any of the claims 3 to 5, wherein the product-related information comprises a recommendation to change the product before the amount of urine and/or faeces present in the absorbent product exceeds the amount that can be absorbed and/or retained by the product.

7. The method according to any of the claims 1 to 6, further comprising the step of predicting a future need of changing the absorbent product (15) based on the concentration of the at least one detected chemical, and to display product-related information on the mobile device indicative of this future need.

8. A mobile monitoring system (1a;1b) for monitoring use of an absorbent product (15) worn by a user (14), **characterised in that** the absorbent product (15) is a male or female incontinence protector, a sanitary pad, a diaper with tape fastener, a pant diaper or a belted diaper, and **in that** the monitoring system (1a;1b) comprises:
- an odour sensor device (3a;3b) capable of being carried by the user (14) and configured to communicate with a mobile device (2), wherein the odour sensor device (3a;3b) is configured to be carried in a pocket of clothing of the user (14), or to be attached to the clothing of the user (14), or wherein the odour sensor device (3a, 3b) is realised in form of an adapter that is detachably connectable to the mobile device (2), and
- a mobile device (2) configured to provide product-related information to the user,
wherein the odour sensor device (3a;3b) is configured to detect at least one chemical indicative of a presence of urine and/or faeces in the product (14), and to transmit a signal indicative of said detection to the mobile device (2), wherein said signal is indicative of the concentration of the at least one detected chemical, the mobile device (2) being configured to obtain capacity information related to the capacity of the absorbent product (15), to receive said signal and to provide product-related information to the user (14) based on both said signal received from the odour sensor device and said capacity information.

9. The mobile monitoring system (1a;1b) according to claim 8, wherein the odour sensor device (3a;3b) and the mobile device (2) are configured to communicate with each other wirelessly.

10. A method in a mobile device (2) for monitoring use of an absorbent product (15) worn by a user (14), **characterised by** the steps of:
- obtaining (S3) capacity information related to the capacity of the absorbent product (15), wherein the absorbent product (15) is a male or female incontinence protector, a sanitary pad, a diaper with tape fastener, a pant diaper or a belted diaper,
- receiving (S6), from an odour sensor device (3a;3b) capable of being carried by the user (14), a signal indicating that the odour sensor device (3a;3b) has detected at least one chemical indicative of a presence of urine and/or faeces in the product (15), wherein said signal is indicative of the concentration of the at least one detected chemical, and wherein the odour sensor device (3a;3b) is configured to be carried in a pocket of clothing of the user (14), or to be attached to the clothing of the user (14), or wherein the odour sensor device (3a, 3b) is realised in form of an adapter that is detachably connectable to the mobile device (2),
- providing (S7) product-related information to the user (14) based on both the signal received from the odour sensor device and said capacity information, and adjusting the content of the product-related information based on said concentration.

11. The method according to claim 10, further comprising the steps of:
- determining (S3a), based on said capacity information, a capacity parameter indicative of the amount of urine and/or faeces that can be absorbed and/or retained by the absorbent product,
- determining (S6a), based on said signal, a voiding parameter indicative of the amount of urine and/or faeces present in the absorbent product,
- comparing (S6b) the capacity parameter and the voiding parameter, and
- providing (S7a) said product-related information based on said comparison.

12. The method according to claim 10 or 11, wherein the capacity information comprises any of, or any combination of:
- the type of the absorbent product (15),
- the absorbency level of the absorbent product (15), and
- the size of the absorbent product (15).

13. The method according to claim 11 or 12, further comprising the step of predicting a future need of changing the absorbent product (15) based on the concentration of the at least one detected chemical, and to display product-related information on the mobile device (2) indicative of this future need.

14. A computer program for monitoring use of an absorbent product worn by a user, **characterised in that** the computer program, when executed by a processor (4) of a mobile device (2) capable of communicating with an odour sensor device (3a;3b), causes the mobile device (2) to perform the method according to any of the claims 10 to 13.

15. A mobile device (2) for monitoring use of an absorbent product (15) worn by a user (14), the mobile device comprising a processor (4), a communication unit (6) for communicating with an odour sensor device (3a;3b), and a storage medium (5) for storing computer programs executable by said processor (4), **characterised in that** the mobile device (2) comprises means for performing the method according to any of the claims 10 to 13.

## Patentansprüche

1. Ein Verfahren zum Überwachen der Verwendung eines absorbierenden Produkts (15), das von einem Benutzer (14) getragen wird,
**dadurch gekennzeichnet, dass** das absorbierende Produkt (15) ein männlicher oder weiblicher Inkontinenzschutz, eine Hygienepad, eine Windel mit Bandbefestigung, eine Hosenwindel oder eine gegurtete Windel ist, und dass das Verfahren die folgenden Schritte umfasst:
- Erhalten (S3) mittels einer mobilen Vorrichtung (2) von Kapazitätsinformationen über die Kapazität des absorbierenden Produkts (15),
- Erfassen (S4) mindestens einer Chemikalie, die auf ein Vorhandensein von Urin und/oder Fäkalien in dem Produkt hinweist, mittels einer Geruchssensorvorrichtung (3a; 3b), die vom Benutzer (14) getragen werden kann und konfiguriert ist, um mit der mobilen Vorrichtung (2) des Benutzers zu kommunizieren, wobei die Geruchssensorvorrichtung (3a;3b) in einer Tasche mit Kleidung des Benutzers (14) getragen oder an der Kleidung des Benutzers (14) befestigt wird, oder wobei die Geruchssensorvorrichtung (3a, 3b) in Form eines Adapters realisiert ist, der lösbar mit der mobilen Vorrichtung (2) verbindbar ist,
- Übertragen (S5) eines Signals, das die Erkennung anzeigt, von der Geruchssensorvorrichtung (3a; 3b) an die mobile Vorrichtung (2), wobei das Signal die Konzentration der mindestens einen erfassten Chemikalie anzeigt,
- Empfangen (S6) des Signals, das die Erkennung anzeigt, in der mobilen Vorrichtung,
- Bereitstellen (S7) produktbezogener Informationen mittels der mobilen Vorrichtung, basierend auf sowohl dem von der Geruchssensorvorrichtung empfangenen Signal als auch den Kapazitätsinformationen.

2. Das Verfahren nach Anspruch 1, ferner umfassend den Schritt des Anpassens des Inhalts der produktbezogenen Informationen basierend auf der Konzentration der mindestens einen erfassten Chemikalie.

3. Das Verfahren nach Anspruch 1, ferner umfassend die Schritte von:
- Bestimmen (S3a), basierend auf den Kapazitätsinformationen, eines Kapazitätsparameters, der die Menge an Urin und/oder Kot anzeigt, die von dem absorbierenden Produkt aufgenommen und/oder zurückgehalten werden kann,
- Bestimmen (S6a), basierend auf dem Signal, eines Entleerungsparameters, der die Menge an Urin und/oder Kot in dem absorbierenden Produkt anzeigt,
- Vergleichen (S6b) des Kapazitätsparameters und des Entleerungsparameters, und
- Bereitstellen (S7a) der produktbezogenen Informationen basierend auf dem Vergleich.

4. Das Verfahren nach Anspruch 3, wobei der Kapazitätsparameter bestimmt wird basierend auf einer von oder einer Kombination von:
- die Art des absorbierenden Produkts (15),
- das Absorptionsvermögen des absorbierenden Produkts (15) und
- die Größe des absorbierenden Produkts (15).

5. Das Verfahren nach Anspruch 3 oder 4, wobei der Entleerungsparameter basierend auf der Konzentration der mindestens einen erfassten Chemikalie bestimmt wird.

6. Das Verfahren nach einem der Ansprüche 3 bis 5, wobei die produktbezogenen Informationen eine Empfehlung zum Wechseln des Produkts umfassen, bevor die Menge an Urin und/oder Kot, die in dem absorbierenden Produkt vorhanden ist, die Menge übersteigt, die von dem Produkt absorbiert und/oder zurückgehalten werden kann.

7. Das Verfahren nach einem der Ansprüche 1 bis 6, ferner umfassend den Schritt des Vorhersagens eines zukünftigen Bedarfs zum Wechseln des absorbierenden Produkts (15) basierend auf der Konzentration der mindestens einen erfassten Chemikalie und zum Anzeigen produktbezogener Informationen auf der mobilen Vorrichtung, die auf diesen zukünftigen Bedarf hinweisen.

8. Ein mobiles Überwachungssystem (1a; 1b) zum Überwachen der Verwendung eines von einem Benutzer (14) getragenen absorbierenden Produkts (15), **dadurch gekennzeichnet, dass** das absorbierende Produkt (15) ein männlicher oder weiblicher Inkontinenzschutz, ein Hygienepad, eine Windel mit Bandbefestigung, eine Hosenwindel oder eine gegurtete Windel ist, und dass das Überwachungssystem (1a; 1b) umfasst:
- eine Geruchssensorvorrichtung (3a; 3b), die vom Benutzer (14) getragen werden kann und konfiguriert ist, um mit einer mobilen Vorrichtung (2) zu kommunizieren, wobei die Geruchssensorvorrichtung (3a; 3b) konfiguriert ist, um in einer Tasche mit Kleidung des Benutzers (14) getragen zu werden oder um an der Kleidung des Benutzers (14) befestigt zu werden, oder wobei die Geruchssensorvorrichtung (3a, 3b) in Form eines Adapters realisiert ist, der lösbar mit der mobilen Vorrichtung (2) verbunden werden kann, und
- eine mobile Vorrichtung (2), die konfiguriert ist, um dem Benutzer produktbezogene Informationen bereitzustellen,
wobei die Geruchssensorvorrichtung (3a, 3b) konfiguriert ist, um mindestens eine Chemikalie zu erfassen, die ein Vorhandensein von Urin und/oder Fäkalien in dem Produkt (14) anzeigt, und um ein Signal zu übertragen, das ein solches anzeigt, an die mobile Vorrichtung (2), wobei das Signal ein Indikator für die Konzentration der mindestens einen erfassten Chemikalie ist, wobei die mobile Vorrichtung (2) konfiguriert ist, um Kapazitätsinformationen zu erhalten, die sich auf die Kapazität des absorbierenden Produkts (15) beziehen, um das Signal zu empfangen und um dem Benutzer (14) produktbezogene Informationen bereitzustellen, die sowohl auf dem von der Geruchssensorvorrichtung empfangenen Signal als auch auf den Kapazitätsinformationen basieren.

9. Das mobile Überwachungssystem (1a;1b) nach Anspruch 8, wobei die Geruchssensorvorrichtung (3a, 3b) und die mobile Vorrichtung (2) konfiguriert sind, um drahtlos miteinander zu kommunizieren.

10. Ein Verfahren in einer mobilen Vorrichtung (2) zum Überwachen der Verwendung eines absorbierenden Produkts (15)15, das von einem Benutzer (14) getragen wird, **gekennzeichnet durch** die Schritte von:
- Erhalten (S3) von Kapazitätsinformationen in Bezug auf die Kapazität des absorbierenden Produkts (15), wobei das absorbierende Produkt (15) ein männlicher oder weiblicher Inkontinenzschutz, eine Hygienepolster, eine Windel mit Klebeband, eine Hosenwindel oder eine gegurtete Windel ist,
- Empfangen (S6) eines Signals von einer Geruchssensorvorrichtung (3a; 3b), die vom Benutzer (14) getragen werden kann, von einem Signal, das anzeigt, dass die Geruchssensorvorrichtung (3a; 3b) mindestens eine Chemikalie erfasst hat, die ein Vorhandensein von Urin und/oder Fäkalien in dem Produkt (15) anzeigt, wobei das Signal die Konzentration der mindestens einen erfassten Chemikalie anzeigt, und wobei die Geruchssensorvorrichtung (3a; 3b) konfiguriert ist, um in einer Tasche mit Kleidung des Benutzers (14) getragen zu werden, oder um an der Kleidung des Benutzers (14) befestigt zu werden, oder worin die Geruchssensorvorrichtung (3a, 3b) in Form eines Adapters realisiert ist, der lösbar mit der mobilen Vorrichtung (2) verbindbar ist,
- Bereitstellen (S7) produktbezogener Informationen für den Benutzer (14) basierend sowohl auf dem von der Geruchssensorvorrichtung empfangenen Signal als auch auf den Kapazitätsinformationen und Einstellen des Inhalts der produktbezogenen Informationen basierend auf der Konzentration.

11. Das Verfahren nach Anspruch 10, ferner umfassend die Schritte von:
- Bestimmen (S3a), basierend auf den Kapazitätsinformationen, eines Kapazitätsparameters, der die Menge an Urin und/oder Kot anzeigt, die von dem absorbierenden Produkt aufgenommen und/oder zurückgehalten werden kann,
- Bestimmen (S6a), basierend auf dem Signal, eines Entleerungsparameters, der die Menge an Urin und/oder Kot in dem absorbierenden Produkt anzeigt,
- Vergleichen (S6b) des Kapazitätsparameters und des Entleerungsparameters, und
- Bereitstellen (S7a) der produktbezogenen Informationen basierend auf dem Vergleich.

12. Das Verfahren nach Anspruch 10 oder 11, wobei die Kapazitätsinformation eine beliebige oder eine beliebige Kombination von:
- die Art des absorbierenden Produkts (15),
- das Absorptionsvermögen des absorbierenden Produkts (15) und
- die Größe des absorbierenden Produkts (15).

13. Das Verfahren nach Anspruch 11 oder 12, ferner umfassend den Schritt des Vorhersagens eines zukünftigen Bedarfs zum Wechseln des absorbierenden Produkts (15) basierend auf der Konzentration der mindestens einen erfassten Chemikalie und des Anzeigens produktbezogener Informationen auf der mobilen Vorrichtung (2), die diesen zukünftigen Bedarf anzeigen.

14. Ein Computerprogramm zum Überwachen der Verwendung eines von einem Benutzer getragenen absorbierenden Produkts, **dadurch gekennzeichnet, dass** das Computerprogramm, wenn es von einem Prozessor (4) einer mobilen Vorrichtung (2) ausgeführt wird, die in der Lage ist, mit einer Geruchssensorvorrichtung (3a, 3b) zu kommunizieren, bewirkt, dass die mobile Vorrichtung (2) das Verfahren nach einem der Ansprüche 10 bis 13 durchführt.

15. Ein mobile Vorrichtung (2) zum Überwachen der Verwendung eines von einem Benutzer (14) getragenen absorbierenden Produkts (15), wobei die mobile Vorrichtung einen Prozessor (4), eine Kommunikationseinheit (6) zum Kommunizieren mit einer Geruchssensorvorrichtung (3a; 3b) und ein Speichermedium (5) zum Speichern von Computerprogrammen, die von dem Prozessor (4) ausführbar sind, umfasst, **dadurch gekennzeichnet, dass** die mobile Vorrichtung (2) Mittel zum Ausführen des Verfahrens nach einem der Ansprüche 10 bis 13 umfasst.

## Revendications

1. Procédé pour surveiller l'utilisation d'un produit absorbant (15) porté par un utilisateur (14),
**caractérisé en ce que** le produit absorbant (15) est une protection contre l'incontinence masculine ou féminine, une serviette hygiénique, une couche avec attache adhésive, une couche-culotte ou une couche avec ceinture, et **en ce que** le procédé comprend les étapes de :
l'obtention (S3), au moyen d'un dispositif mobile (2), d'informations de capacité relatives à la capacité du produit absorbant (15),
la détection (S4), au moyen d'un dispositif de capteur d'odeur (3a ; 3b) pouvant être transporté par l'utilisateur (14) et configuré pour communiquer avec ledit dispositif mobile (2) de l'utilisateur, d'au moins une substance chimique indicative d'une présence d'urine et/ou de fèces dans le produit, le dispositif de capteur d'odeur (3a ;3b) étant transporté dans une poche de vêtement de l'utilisateur (14), ou fixé sur le vêtement de l'utilisateur (14), ou le dispositif de capteur d'odeur (3a ; 3b) étant réalisé sous la forme d'un adaptateur qui peut être connecté de manière amovible au dispositif mobile (2),
la transmission (S5), depuis ledit dispositif de capteur d'odeur (3a ; 3b) audit dispositif mobile (2), d'un signal indicatif de ladite détection, ledit signal étant indicatif de la concentration de l'au moins une substance chimique détectée,
la réception (S6), dans ledit dispositif mobile, du signal indicatif de la détection,
la fourniture (S7), au moyen dudit dispositif mobile, d'informations associées au produit sur la base à la fois dudit signal reçu depuis le dispositif de capteur d'odeur et desdites informations de capacité.

2. Procédé selon la revendication 1, comprenant en outre l'étape d'ajustement du contenu des informations associées au produit sur la base de la concentration de l'au moins une substance chimique détectée.

3. Procédé selon la revendication 1, comprenant en outre les étapes de :
- détermination (S3a), sur la base desdites informations de capacité, d'un paramètre de capacité indicatif de la quantité d'urine et/ou de fèces qui peut être absorbée et/ou retenue par le produit absorbant,
- détermination (S6a), sur la base dudit signal, d'un paramètre d'excrétion indicatif de la quantité d'urine et/ou de fèces présentes dans le produit absorbant,
- comparaison (S6b) du paramètre de capacité et du paramètre d'excrétion, et
- fourniture (S7a) desdites informations associées au produit sur la base de ladite comparaison.

4. Procédé selon la revendication 3, le paramètre de capacité étant déterminé sur la base de l'une quelconque de, ou de l'une quelconque d'une combinaison de :
- le type du produit absorbant (15),
- le niveau d'absorption du produit absorbant (15), et
- la taille du produit absorbant (15).

5. Procédé selon la revendication 3 ou 4, le paramètre d'excrétion étant déterminé sur la base de la concentration de l'au moins une substance chimique détectée.

6. Procédé selon l'une quelconque des revendications 3 à 5, les informations associées au produit comprenant une recommandation de changer le produit avant que la quantité d'urine et/ou de fèces présentes dans le produit absorbant dépasse la quantité qui peut être absorbée et/ou retenue par le produit.

7. Procédé selon l'une quelconque des revendications 1 à 6, comprenant en outre l'étape de prévision d'un besoin futur de changer le produit absorbant (15) sur la base de la concentration de l'au moins une substance chimique détectée, et d'affichage des informations associées au produit sur le dispositif mobile, indicatives de ce besoin futur.

8. Système de surveillance mobile (1a ; 1b) pour surveiller l'utilisation d'un produit absorbant (15) porté par un utilisateur (14), **caractérisé en ce que** le produit absorbant (15) est une protection contre l'incontinence masculine ou féminine, une serviette hygiénique, une couche avec attache adhésive, une couche-culotte ou une couche avec ceinture, et **en ce que** le système de surveillance (1a ; 1b) comprend :
- un dispositif de capteur d'odeur (3a ; 3b) pouvant être transporté par l'utilisateur (14) et configuré pour communiquer avec un dispositif mobile (2), le dispositif de capteur d'odeur (3a ; 3b) étant configuré pour être transporté dans une poche de vêtement de l'utilisateur (14), ou pour être fixé sur le vêtement de l'utilisateur (14), ou le dispositif de capteur d'odeur (3a, 3b) étant réalisé sous la forme d'un adaptateur qui est connecté de façon amovible au dispositif mobile (2), et
- un dispositif mobile (2) configuré pour fournir des informations associées au produit à l'utilisateur, le dispositif de capteur d'odeur (3a ; 3b) étant configuré pour détecter au moins une substance chimique indicative d'une présence d'urine et/ou de fèces dans le produit (14), et pour transmettre un signal indicatif de ladite détection au dispositif mobile (2), ledit signal étant indicatif de la concentration de l'au moins une substance chimique détectée, le dispositif mobile (2) étant configuré pour obtenir des informations de capacité associées à la capacité du produit absorbant (15), pour recevoir ledit signal et fournir des informations associées au produit à l'utilisateur (14) sur la base à la fois dudit signal reçu depuis le dispositif de capteur d'odeur et desdites informations de capacité.

9. Système de surveillance mobile (1a ; 1b) selon la revendication 8, le dispositif de capteur d'odeur (3a ; 3b) et le dispositif mobile (2) étant configurés pour communiquer entre eux sans fil.

10. Procédé dans un dispositif mobile (2) pour surveiller l'utilisation d'un produit absorbant (15) porté par un utilisateur (14), **caractérisé par** les étapes de :
- l'obtention (S3) d'informations de capacité associées à la capacité du produit absorbant (15), le produit absorbant (15) étant une protection contre l'incontinence masculine ou féminine, une serviette hygiénique, une couche avec attache adhésive, une couche-culotte ou une couche avec ceinture,
- la réception (S6), depuis un dispositif de capteur d'odeur (3a ; 3b) pouvant être transporté par l'utilisateur (14), d'un signal indiquant que le dispositif de capteur d'odeur (3a ; 3b) a détecté au moins une substance chimique indicative d'une présence d'urine et/ou de fèces dans le produit (15), ledit signal étant indicatif de la concentration de l'au moins une substance chimique détectée, et le dispositif de capteur d'odeur (3a ;3b) étant configuré pour être transporté dans une poche de vêtement de l'utilisateur (14), ou pour être fixé sur le vêtement de l'utilisateur (14), ou le dispositif de capteur d'odeur (3a, 3b) étant réalisé sous la forme d'un adaptateur qui peut être connecté de manière amovible au dispositif mobile (2),
- la fourniture (S7) d'informations associées au produit à l'utilisateur (14) sur la base à la fois du signal reçu depuis le dispositif de capteur d'odeur et desdites informations de capacité, et l'ajustement du contenu des informations associées au produit sur la base de ladite concentration.

11. Procédé selon la revendication 10, comprenant en outre les étapes de :
- détermination (S3a), sur la base desdites informations de capacité, d'un paramètre de capacité indicatif de la quantité d'urine et/ou de fèces qui peut être absorbée et/ou retenue par le produit absorbant,
- détermination (S6a), sur la base dudit signal, d'un paramètre d'excrétion indicatif de la quantité d'urine et/ou de fèces présentes dans le produit absorbant,
- comparaison (S6b) du paramètre de capacité et du paramètre d'excrétion, et
- fourniture (S7a) desdites informations associées au produit sur la base de ladite comparaison.

12. Procédé selon la revendication 10 ou 11, les informations de capacité comprenant l'une quelconque de, ou l'une quelconque d'une combinaison de :
- le type du produit absorbant (15),
- le niveau d'absorption du produit absorbant (15), et
- la taille du produit absorbant (15).

13. Procédé selon la revendication 11 ou 12, comprenant en outre l'étape de prévision d'un besoin futur de changer le produit absorbant (15) sur la base de la concentration de l'au moins une substance chimique détectée, et d'affichage des informations associées au produit sur le dispositif mobile (2), indicatives de ce besoin futur.

14. Programme informatique pour surveiller l'utilisation d'un produit absorbant porté par un utilisateur, **caractérisé en ce que** le programme informatique, lorsqu'il est exécuté par un processeur (4) d'un dispositif mobile (2) pouvant communiquer avec un dispositif de capteur d'odeur (3a ; 3b), amène le dispositif mobile (2) à réaliser le procédé selon l'une quelconque des revendications 10 à 13.

15. Dispositif mobile (2) pour surveiller l'utilisation d'un produit absorbant (15) porté par un utilisateur (14), le dispositif mobile comprenant un processeur (4), une unité de communication (6) pour communiquer avec un dispositif de capteur d'odeur (3a ; 3b), et un support de stockage (5) pour stocker des programmes informatiques exécutables par ledit processeur (4), **caractérisé en ce que** le dispositif mobile (2) comprend des moyens pour réaliser le procédé selon l'une quelconque des revendications 10 à 13.
